# EUROPEAN PATENT APPLICATION

(11) **EP 4 516 392 A1**
(43) Date of publication of application: **05.03.2025**
(21) Application number: 23195207.8
(22) Date of filing: 04.09.2023
(51) Int. Cl.: B01J 19/00, B01J 19/24

(54) **PEPTIDES, PEPTIDIC NUCLEIC ACIDS (PNA) AND OLIGONUCLEOTIDES SYNTHESIS ASSISTED BY MEMBRANE EXTRACTION**

(71) Applicant: VITO NV, 2400 Mol (BE); Alma Mater Studiorum Universita di Bologna, 40126 Bologna (IT)
(72) Inventor: ORMEROD, Dominic, 2400 Mol (BE); SO, Wing Ho, 2400 Mol (BE); GRATECAT, Izaskun, 2400 Mol (BE); CABRI, Walter, 40126 Bologna (IT); TOLOMELLI, Alessandra, 40126 Bologna (IT); FERRAZZANO, Lucia, I-40126 Bologna (IT)
(74) Representative: Arnold & Siedsma

(57) **Abstract**

The present invention is related to a system and method for peptide and oligonucleotide ("tide") manufacture, making use of reactor with discriminating membrane technology to separate out byproducts and excess reagents from the reaction mixture. The discriminating membrane technology is based on the application of a membrane which is configured to act as a solid phase extraction membrane between a reaction chamber and an extraction chamber present within the reactor with an affinity for reagents and by-products present in the reaction chamber.

## Description

### Technical field

The present invention is related to a system and method for peptide and oligonucleotide ("tide") manufacture, making use of reactor with discriminating membrane technology to separate out byproducts and excess reagents from the reaction mixture using green organic solvents.

The discriminating membrane technology is based on the application of a membrane which is configured to act as a solid phase extraction membrane between a reaction chamber and an extraction chamber present within the reactor with an affinity for reagents and by-products present in the reaction chamber thereby limiting the amount of organic solvents and water necessary to complete the coupling-deprotection sequence that need to be iterated.

### Background art

Peptide, PNA and oligonucleotide chemical synthesis are iterative technologies like Solid Phase Peptide Synthesis (SPPS) or Liquid Phase Peptide Synthesis (LPPS) (Green Chemistry 2022, 24, 975-1020). The membrane based technology can be applied to fluorenylmethoxycarbonyl (Fmoc) LPPS for peptides and PNAs (WO2016188835A1; US8664357; Angew. Chem. Int. Ed. 2021, 60, 7786 - 7795) and to convergent fragments base synthesis (Org. Process Res. Dev. 2021, 25, 1628-1636). The Liquid Phase Oligonucleotide Synthesis (LPOS) using membranes to eliminate impurities and side products was described by Livingston Org. Process Res. Dev. 2016, 20, 1439-1452.

The use of a separation barrier between a reaction chamber and supply chamber to separate out the byproducts and excess reagents is known in the art of tide synthesis via LPPS and LPOS. In those solutions the membrane acts simply as a physical barrier with a molecular cut-off value to retain the reaction product in the reaction chamber.

The barriers used in such systems may be provided in a variety of ways, such as porous membranes, porous particles, hollow fibers, fritted discs, or the like. The porous barrier may be a single or multiple layer, may be in the form of sheets, particles, hollow fibers, tubes, or the like. Numerous configurations may be designed which allow for maintenance of the desired components in the reaction region with the removal of the undesired components from the reaction region. By providing for a source of the low molecular weight reactants to the reaction region, so as to replenish or augment the depletion of such components during the course of the reaction, while at the same time removing the low molecular weight products of the reaction, one can greatly extend the course of the period of expression and provide for high molar ratios of protein or oligonucleotide product to template present in the reaction medium.

In its simplest configuration these systems consist of a membrane bag containing the reaction region, while retaining a solution outside the membrane which provides for the desired level of the lower molecular weight reaction components in the reaction region. Thus, by exchange across the membrane, the lower molecular weight products produced by the reaction will be continuously dialyzed into the external solution, while the reaction components will be continuously replenished in the reaction region.A down-size of these systems applied to peptides and oligonucleotide synthesis is that large volumes of the external solution are required to keep a proper flux across the barrier and keep the amount of low molecular weight products within the reaction region to a minimum; The process mass intensity (PMI) that is the total process mass kg/kg of product is dramatically increasing with respect to the solid phase technology, increasing also the process costs.

Another drawback is the increasing of process time. Again in comparison to solid phase technology, the length of time required to perform this operation is longer than that required to wash impurities away from a growing peptide, oligonucleotide or PNA anchored onto a solid phase resin. This is principally due to the fact that the volume of solvent required to wash impurities out of a system using the membrane process of Livingston is greater much than that required in solid phase synthesis and as such unless prohibitively large membrane surface area's are used, the operation time will be quite long.

Thus, it would be desirable to develop an improved system for tide synthesis that can operated at a low trans-membrane pressure and that produces less waste solution, with the target to have a PMI lower than the corresponding SPPS and competitive process time

### Summary of invention

According to a first aspect of the invention, there is therefore provided a liquid phase tide synthesis system having a housing (1) comprising a reaction chamber (2) and an extraction chamber (3) separated from each other by a membrane (4), characterized in that the membrane is configured to act as a solid phase extraction membrane between the reaction chamber and extraction chamber with an affinity for reagents and by-products present in the reaction chamber. Surprisingly and in contrast to the currently available technology the PMI of the coupling-deprotection is consistently below 80 that represent the limit of the SPPS and Green Solid-Phase Peptide Synthesis (GSPPS) in particular.

The technology can be applied to several carboxylic acid terminal groups. Simple esters, like tBu, benzyl or trimethoxybenzyl (Tmob) or otherhigh molecular weight anchors, such as bis(4-methoxyphenyl)methylamine, (2,5-bis((3,7,11,15-tetramethylhexadecyl)oxy)phenyl)methanol, and (2,5-bis((3,7,11,15-tetramethylhexadecyl)oxy)phenyl)methylamine. In this case the terminal functional group can be an ester or an amide (Angew. Chem. 2017, 129, 7911 -7915; EP2612845B1; US8093435B2; Org. Process Res. Dev. 2019, 23, 2576-2581; Angew. Chem. Int. Ed. 2021, 60, 7786 - 7795). Several coupling systems can be used.

The amino protective group Fmoc it is removed under basic conditions typically in the presence of an organic amine, such as for example but not limited to piperidine, pyrrolidine, diethyl amine, and the like.

The separation technology can be applied after every single step or after the coupling-deprotection sequence.

In an embodiment of the invention, the membranes are polymeric or ceramic nanofiltration membranes, in particular ceramic nanofiltration membranes with a Molecular Weight Cut-Off (MWCO) of less than 900.

The most common ceramic membranes are made of Al, Si, Ti or Zr oxides, with Ti and Si being more stable than Al or Si oxides. In some less frequent cases, Sn or Hf are used as base elements. Each oxide has a different surface charge in solution, with different surface characteristics in sense of surface polarity. Other membranes can be composed of mixed oxides of two of the previous elements, or are established by some additional compounds present in minor concentration. In a particular embodiment the membrane is a ceramic TiO₂ membrane with a thickness from about 1 to 5 nm.

In an embodiment the system further comprises a feed section (5) and an extraction section (6) wherein the feed section is configured circulate a reaction solution (7) across the reaction chamber and wherein the extraction section is configured to circulate an extraction solution (8) across the extraction chamber.

In an embodiment of the invention, the polarity of the reaction solution is such that the membrane (4) separating the reaction chamber from the extraction chamber, is saturated with the reaction solution. Expressed differently, the membrane surface polarity matches with the polarity of the reaction solution, such that the membrane is saturated with the later. The polarity of the reaction solution is primarily dependent of the reaction solvent being used. Consequently, in a particular embodiment the polarity of the reaction solvent is such that the membrane (4) separating the reaction chamber from the extraction chamber, is saturated with the reaction solvent.

In an embodiment of the process the extraction can be performed after each step of coupling and deprotection or after the sequence coupling-deprotection.

In an embodiment of the system according to the invention, the reaction solvent is polar aprotic and any kind of coupling reagent as well as FMOC deprotection system known to the person skilled in the art, in particular selected from ethyl acetate, anisole or DMF.

For the extraction solution (8) it has been observed that best results in circulating the reaction solution and the extraction solution are achieved when the extraction solution is a reactive extraction solution, in that it reacts with the reagents and/or by-products crossing the membrane. The reactivity of the extraction solution is primarily dependent of the extraction solvent being used. Consequently, in a particular embodiment the extraction solvent is a reactive extraction solvent, in that it reacts with the reagents and/or by-products crossing the membrane. If for example an amine is to be removed, the extraction solution is reactive when it is an acidic extraction solution. Being acidic it would protonate the extracted amine and prevent it from reverse crossing the membrane, i.e. from the extraction chamber into the reaction chamber. Though reactive extraction is the most efficient, it is not obliged. A difference in solubility between the feed phase and the extraction phase can also be used, though this is typically less efficient than reactive extraction.

In an embodiment of the invention, the extraction solvent is selected from an aqueous acid solution or ethanol. Where an aqueous acid solution is a reactive extraction solution in the context of the present invention, ethanol can also be used and is based on differences in the solubility of the reaction secondary products in the feed solvent and ethanol. As explained above this isn't reactive extraction and tends to be less efficient than the aqueous acid extraction.

### Brief description of the figures

Various aspects of the invention will now be discussed in greater detail with reference to the appended drawings, with the same reference numerical illustrating the same attributes.
Figure 1 - Schematic representation of a liquid phase tide synthesis system according to the invention
Figure 2 - Ln (1 -extraction efficiency) over time of the TMB-ester (full lines) and the DBF-piperidine adduct (dotted lines) respectively across the Puramem-selective membrane (squares) and the ceramic PS-TiO2 membrane (circles).

### Description of embodiments

### Cross reference numbers

1. Housing
2. Reaction Chamber
3. Extraction Chamber
4. Membrane
5. Feed Section
6. Extraction Section
7. Reaction Mixture, also referred to as Reaction Solution
8. Extraction Mixture, also referred to as Extraction Solution
9. Reaction Supply Container
10. Reaction Chamber Inlet
11. Reaction Chamber Outlet
12. Filling Opening
13. Extraction Supply Container
14. Extraction Chamber Outlet
15. Extraction Chamber Inlet
16. Reaction Solution Supply Means
17. Reaction Solution Pump
18. Reaction Solution Feed Line
19. Extraction Solution Supply Means
20. Extraction Solution Pump
21. Extraction Solution Feed Line

Peptides, oligonucleotides and peptide nucleic acids are important biomolecules collectively referred to as "tides". As mentioned hereinbefore, it is an object of the present invention to provide a system for improved liquid phase tide synthesis tide synthesis techniques to cost-effectively achieve quality and yield of tides while reducing batch variability.

To said extend the present inventors have developed a novel system to greatly reduce the amount of extraction solution to achieve purity specifications comparable to solid phase tide synthesis, but requiring large volumes of solvents to wash away impurities from the reaction product.

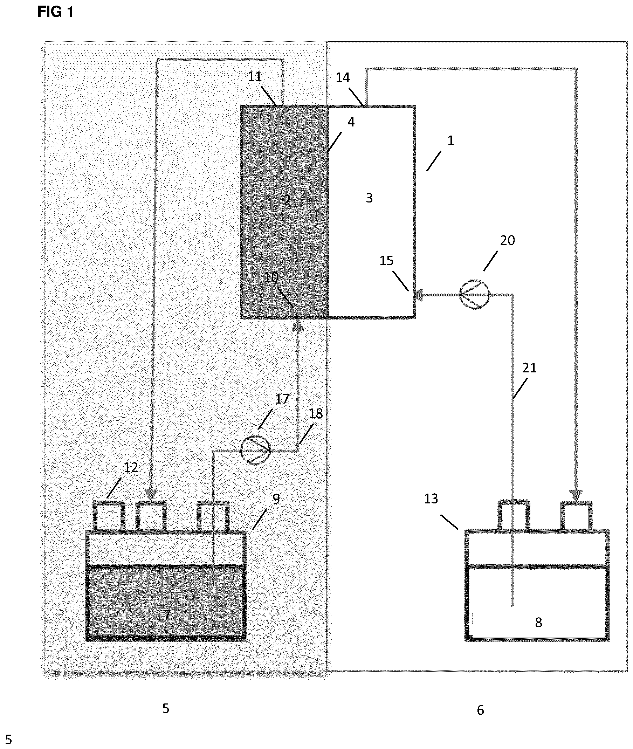

With reference to Figure 1, the liquid phase tide synthesis system is based on the presence of a housing (1) comprising a reaction chamber (2) and an extraction chamber (3) separated from each other by a membrane (4). In existing systems, such as for example described in US patent US 6,670,173 the membrane is a semi-permeable membrane that allows a bi-directional exchange of products and liquids between the chambers, with a selectivity that is based on size exclusion. With these semi-permeable membranes, transport across the membrane is primarily driven by a concentration gradient for reagents and/or by-products between the reaction solution and the extraction solution. This explains why large volumes of extraction solution are required to achieve the desired purity of the reaction product in the reaction chamber.

It has been recognized by the present inventors that this problem can be addressed by using a different type of membrane to separate the reaction chamber from the extraction chamber in a liquid phase tide synthesis system. Instead of a naive semi-permeable membrane, a membrane is used that acts as a solid phase extraction membrane with an affinity for the reagents and by-products present in the reaction chamber. Expressed differently, compared to the semi-permeable membranes of the prior art, the solid phase extraction membrane used in the system according to the invention combines size exclusion with phase extraction to enhance a directional transport of reagents and by-products from the reaction chamber into the extraction chamber.

To said effect the membranes used in the present invention are selected to have a surface polarity that matches the polarity of the reaction solution, to have an affinity for the reagents and/or by-products, and to have a polarity contrary to the polarity of the extraction solution. Doing so, the membrane will be saturated with the reaction solution and pull the excess reagents and by-products from the reaction chamber across the membrane. Given the difference in polarity with the extraction solution, the latter will not penetrate into the membrane, thus creating an interface at the surface of the membrane to selectively extract the reagents and by-products from the reaction chamber into the extraction chamber.

In having a combined effect on the aforementioned transport of excess reagents and by-products, it has also been observed that use of a solid phase extraction membrane also prevents membrane fouling.

In a particular embodiment the solid phase extraction membranes used are either ceramic of polymeric nanofiltration membranes, preferably ceramic of polymeric nanofiltration membranes with a Molecular Weight Cut-Off (MWCO) of less than 900, more in particular ceramic nanofiltration membranes, even more in particular ceramic nanofiltration membranes with a Molecular Weight Cut-Off (MWCO) of less than 900.

**Table 1- Preferred membranes**

| **membrane** | **material** | **MWCO** | **Surface characteristics** |
|---|---|---|---|
| 0.9 nm TiO₂ | Ceramic | 450 | polar |
| 1.0 nmTiO₂ | | 750 | |
| 0.9 nm C₁ TiO₂ | ceramic | 450 | intermediate |
| Puramem performance | polymeric | Not reported | low |
| 5 nm PS TiO₂ | ceramic | <900 | |

The system further comprises a feed section (5) and an extraction section (6). In the feed section a reaction mixture / solution (7) is circulated across the reaction chamber and in the extraction section an extraction mixture / solution (8) is circulated across the extraction chamber. In the embodiment shown in Figure 1, a reaction mixture is fed from a reaction supply container (9) by means of a reaction chamber inlet (10) into the reaction chamber. Via a reaction chamber outlet (11) the reaction solution is recirculated into the reaction supply container. To replenish spent reagents, the container is equipped with an additional filling opening (12).

At the extraction section (6) a similar configuration exists. An extraction solution is fed from an extraction supply container (13) into the extraction chamber. Via an extraction chamber outlet (14) the extraction solution is recirculated into the extraction supply container through an extraction chamber inlet (15). In the shown embodiment, the extraction section consists of a closed loop. As mentioned before, combining the extraction solution with a solid phase extraction membrane with affinity for the reaction solution comprising the reagents and by-products, enhances the transport of excess reagents and by-products across the membrane into the extraction solution and enables a closed loop of the extraction solution at the extraction section, in particular in said instance where the extraction solution is a reactive extraction solution.

As used herein, a reactive extraction solution, is chemical species constituting, or present in, the extraction solution that reacts with the extracted chemical species, in the instant application with at least the excess reagents and preferably also with possible by-products. Reactive liquid extraction processes are known, such as the extraction of carboxylic acids with amine extractants, or vice-versa (Tamada, J. A.; Kertes, A. S.; King, C. J. "Extraction of carboxylic acids with amine extractants. 1. equilibria and law of mass action modeling". Ind. Eng. Chem. Res. 29, pages 1319-1326, 1990).

### Numbered embodiments of the invention

1. A liquid phase tide synthesis system having a housing (1) comprising a reaction chamber (2) and an extraction chamber (3) separated from each other by a membrane (4), characterized in that the membrane is configured to act as a solid phase extraction membrane between the reaction chamber and extraction chamber.
2. The liquid phase tide synthesis system of embodiment 1, wherein the membrane is configured to act as a solid phase extraction membrane between the reaction chamber and extraction chamber with an affinity for reagents and optionally also for by-products present in the reaction chamber.
3. The liquid phase tide synthesis system of embodiments 1 or 2, wherein the membrane is a polymeric or ceramic nanofiltration membrane, in particular a ceramic nanofiltration membrane with a Molecular Weight Cut-Off (MWCO) of less than 900.
4. The liquid phase tide synthesis system of embodiment 3, wherein membrane is selected from the membranes of Table 1.
5. The liquid phase tide synthesis system according to any one of the preceding embodiments, wherein the system further comprises a feed section (5) and an extraction section (6) wherein the feed section is configured to circulate a reaction solution (7) across the reaction chamber and wherein the extraction section is configured to circulate an extraction solution (8) across the extraction chamber.
6. The liquid phase tide synthesis system according to embodiment 5, wherein the circulation of the extraction solution (8) across the extraction chamber is a closed loop.
7. The liquid phase tide system according to embodiment 5, wherein the membrane has a membrane surface polarity such that the membrane (4) separating the reaction chamber from the extraction chamber, is saturated with the reaction solvent.
8. The liquid phase tide system according to embodiment 5, wherein the reaction solution has a polarity such that the membrane (4) separating the reaction chamber from the extraction chamber, is saturated with the reaction solution.
9. The liquid phase tide system according to embodiment 8, wherein the reaction solution comprises a reaction solvent that has a polarity such that the membrane (4) separating the reaction chamber from the extraction chamber, is saturated with the reaction solvent.
10. The liquid phase tide system according to embodiment 9, wherein the reaction solvent is selected from ethyl acetate, anisole or DMF.
11. The liquid phase tide synthesis system according to embodiment 5, wherein the feed section comprises a reaction supply container (9) configured to feed the reaction solution via a reaction chamber inlet (10) into the reaction chamber.
12. The liquid phase tide synthesis system according to embodiments 5 or 11,
   wherein the feed section further comprises reaction solution supply means (16) such as a reaction solution pump (17) in a reaction solution feed line (18).
13. The liquid phase tide synthesis system according to embodiments 11 or 12,
   wherein the reaction chamber comprises a reaction chamber outlet (11) to recirculate the reaction solution to the reaction supply container.
14. The liquid phase tide synthesis system according to any one of embodiments 11 to 13, wherein the reaction supply container comprises a filling opening (12).
15. The liquid phase tide synthesis system according to embodiment 5, wherein the extraction section comprises an extraction supply container (13) configured to feed the extraction solution via an extraction chamber inlet (15) into the reaction chamber.
16. The liquid phase tide synthesis system according to embodiments 5 or 15,
   wherein the extraction section further comprises extraction solution supply means (19) such as a reaction solution pump (20) in a reaction solution feed line (21).
17. The liquid phase tide synthesis system according to embodiments 15 or 16,
   wherein the reaction chamber comprises an extraction chamber outlet (14) to recirculate the extraction solution to the extraction supply container.
18. The liquid phase tide system according to any one of the preceding embodiments, wherein the membrane (4) is a ceramic TiO₂ membrane with a thickness from about 1 to 5 nm.
19. The liquid phase tide system according to any one of embodiments 5 to 18,
   wherein the extraction solution is a reactive extraction solution.
20. The liquid phase tide system according to embodiment 19, wherein the extraction solution is selected from an aqueous acid solution or ethanol.
21. A liquid phase tide synthesis method comprising the use of a solid phase extraction membrane (4) to separate and extract reagents and optionally also by-products from a reaction solution into an extraction solution.
22. The liquid phase tide synthesis method of embodiment 21, wherein the solid phase extraction membrane (4) has an affinity for reagents and optionally also for by-products present in the reaction chamber.
23. The liquid phase tide synthesis method of embodiments 21 or 22, wherein the membrane is a polymeric or ceramic nanofiltration membrane, in particular a ceramic nanofiltration membrane with a Molecular Weight Cut-Off (MWCO) of less than 900.
24. The liquid phase tide synthesis method of embodiment 23, wherein the membrane is selected from the membranes of Table 1..
25. The liquid phase tide synthesis method according to any one of embodiments 21 to 24, wherein the membrane has a membrane surface polarity such that the the solid phase extraction membrane (4), separating the reaction solution from the extraction solution is saturated with the reaction solvent.
26. The liquid phase tide synthesis method according to embodiment 21, wherein the reaction solution has a polarity such that the solid phase extraction membrane (4) separating the reaction solution from the extraction solution, is saturated with the reaction solution.
27. The liquid phase tide synthesis method according to embodiment 26, wherein the reaction solution comprises a reaction solvent that has a polarity such that the solid phase extraction membrane (4) separating the reaction solution from the extraction solution, is saturated with the reaction solvent.
29. The liquid phase tide synthesis method according to embodiment 27, wherein the reaction solvent is selected from ethyl acetate, anisole or DMF.
30. The liquid phase tide synthesis method according to embodiment 21, wherein the extraction solution is a reactive extraction solution for the reagents and optionally also for the by-products from the reaction solution.
31. The liquid phase tide synthesis method according to embodiment 30, wherein the extraction solution is selected from an aqueous acid solution or ethanol.
32. The liquid phase tide synthesis method according to embodiment 30, wherein the solid phase extraction membrane (4) is a ceramic TiO₂ membrane with a thickness from about 1 to 5 nm.
33. Use of a solid phase extraction membrane (4) as herein provided in its different embodiments, in a liquid phase tide synthesis method and/or system.

### Examples

In case of peptide synthesis each chain step consists of reacting an amino acid (AAₓ) or short chain peptide with C-terminal protection (C-PG), typically trimethoxybenzyl (Tmob) schematically shown in scheme 1 below, with an amino acid (AAₓ₊₁) with a protecting group on the N-terminal of the amino acid (N-PG), typically the Fmoc protecting group, schematically shown below.

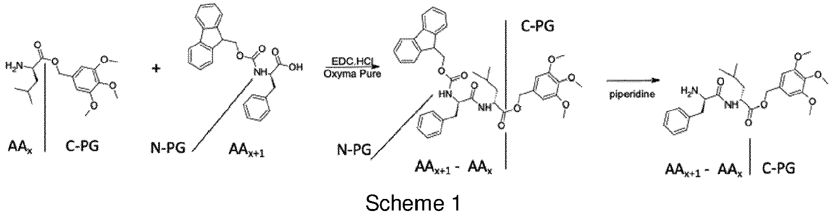

Conceptually the extraction could be carried out at either directly after chain elongation with the N-terminal protection still on the molecule, after removal of the N-terminal protection as suggested in the overall synthesis sequence above or after both. Experiments have shown the process to work best after removal of the N-terminal protection i.e. only one membrane operation per chain elongation step is then required.

### Example 1

In each of the elongation steps piperidine is added to remove the N-terminal protecting group, yielding the AAₓ - C-PG in the above scheme as a starting product. For the subsequent synthesis it is important that the DBF-piperidine adduct, see scheme 2 below, is removed from the reaction medium

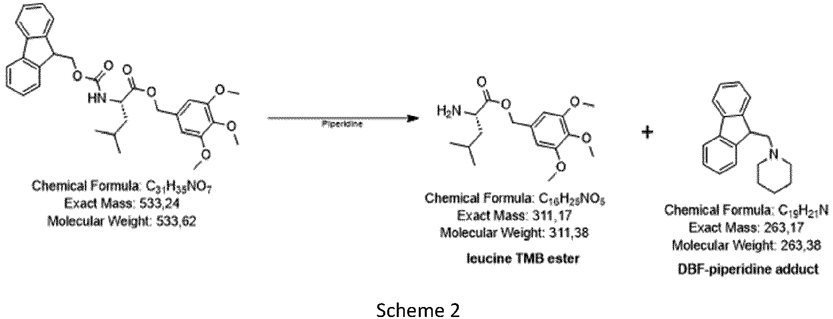

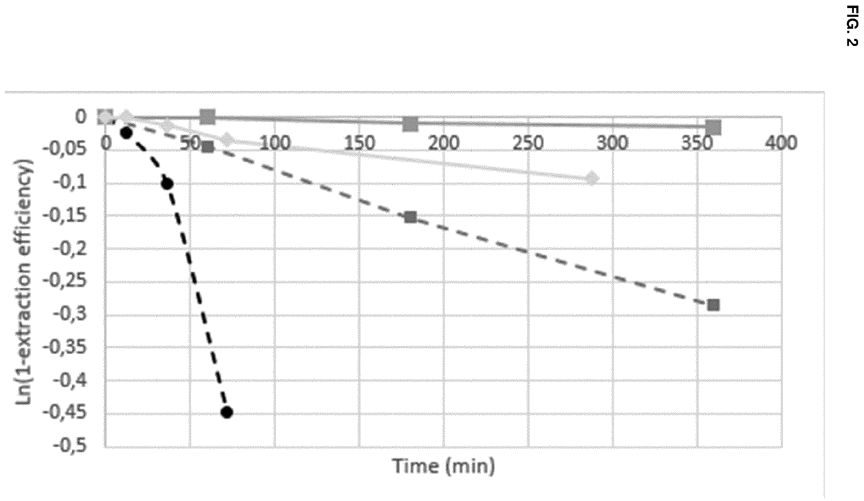

Using different membrane and feed solvent it has been found that extraction efficiency is dependent on the membrane and feed solvent being used but in general low polarity membranes are working best in combination with reactive extraction using aqueous acid as extractant. The rate of extraction is further significantly increased in case ceramic membranes with a low polarity (e.g., PS-TiO₂ below) are being used. Figure 2 compares the extraction efficiency between the Puramem-selective membrane and the ceramic PS-TiO₂ membrane. Both do well retain the TMB-ester, but the PS-TiO₂ membrane is obviously more efficient in a fast removal of the DBF-piperidine adduct.

### Results summary - Table 1:

| Membrane | Feed solvent* | Duration of extraction (min) | % in retentate | |
|---|---|---|---|---|
| | | | Leucine-TMB | DBF-piperidine adduct |
| Puramem-selective | anisole | 1440 | 95.8 | 0.0 |
| 5 nm PMMA-TiO₂ | anisole | 720 | 98.4 | 92.5 |
| 5 nm PS-TiO₂ | DMF | 288 | 91.1 | 0.0 |
| * Extraction solvent in all cases is aqueous HCl at pH 3 | | | | |

In the Table 1, the PMMA is a more polar membrane than the polystyrene. This can be seen in water permeability of the two membranes:
5 nm PMMA TiO₂ water permeability = 6.9 Um*h*bar
5 nm PS TiO₂ water permeability = 0.32 L/m*h*bar

Under the conditions, the solutes used in feed are as yet not protonated and are therefore of quite low polarity compared to polar membranes. Due to its higher polarity when compared to the PS-TiO₂ membrane, the PMMA membrane is acting more like a solvent than a sieve and so, the higher polarity of this membrane ensures limited or at least lower affinity of the membrane for the solute intended to go across the membrane. Thus, with polar membranes transport is retarded, and with an unmodified TiO₂ membrane nothing went across the membrane either. These results thus indicate that low-polarity membranes work best with the reactive extraction solution mentioned above.

Expressed differently, both the solutes and the membranes have in the reaction conditions used a low polarity, as a consequence the membranes are not the reactive element in the extraction of the solutes. The reactive element in the extraction of the solutes is the extraction solution which being acid will protonate any base and prevent any possible reverse transport over the membrane.

For the solvent, and as already explained herein before, the membrane and the reaction solution are matched based on the like dissolves like approach, such that the reaction solution will wet the membrane and pull the excess reagents and by-products from the reaction chamber into and across the membrane. The solvent used in the reaction solution should accordingly dissolve all the components, and even when taken into isolation fit with the aforementioned like dissolves like approach with a wetting of the membrane.

## Claims

**1.** A liquid phase tide synthesis system having a housing (1) comprising a reaction chamber (2) and an extraction chamber (3) separated from each other by a membrane (4), **characterized in that** the membrane is configured to act as a solid phase extraction membrane between the reaction chamber and extraction chamber with an affinity for reagents and optionally also for by-products present in the reaction chamber.

**2.** The liquid phase tide synthesis system according to claim 1, wherein the system further comprises a feed section (5) and an extraction section (6) wherein the feed section is configured to circulate a reaction solution (7) across the reaction chamber and wherein the extraction section is configured to circulate an extraction solution (8) across the extraction chamber.

**3.** The liquid phase tide synthesis system according to claim 2, wherein the circulation of the extraction solution (8) across the extraction chamber is a closed loop.

**4.** The liquid phase tide system according to claim 2, wherein the membrane has a membrane surface polarity such that the membrane (4) separating the reaction chamber from the extraction chamber, is saturated with the reaction solvent.

**5.** The liquid phase tide system according to claim 4, wherein the reaction solvent is selected from ethyl acetate, anisole or DMF.

**6.** The liquid phase tide synthesis system according to claim 2, wherein the feed section comprises a reaction supply container (9) configured to feed the reaction solution via a reaction chamber inlet (10) into the reaction chamber.

**7.** The liquid phase tide synthesis system according to claim 6, wherein the reaction supply container comprises a filling opening (12).

**8.** The liquid phase tide system according to any one of the preceding claims,
wherein the membrane (4) is a ceramic TiO₂ membrane with a thickness from about 1 to 5 nm.

**10.** The liquid phase tide system according to any one of claims 2 to 9, wherein the extraction solution is a reactive extraction solution.

**11.** The liquid phase tide system according to claim 10, wherein the extraction solution is selected from an aqueous acid solution or ethanol.

**12.** A liquid phase tide synthesis method comprising the use of a solid phase extraction membrane (4) to separate and extract reagents and optionally also by-products from a reaction solution into an extraction solution, and wherein the solid phase extraction membrane (4) has an affinity for reagents and optionally also for by-products present in the reaction chamber.

**13.** The liquid phase tide synthesis method according to claim 12, wherein the membrane has a membrane surface polarity such that the solid phase extraction membrane (4), separating the reaction solution from the extraction solution is saturated with the reaction solvent.

**14.** The liquid phase tide synthesis method according to claim 13, wherein the reaction solvent is selected from ethyl acetate, anisole or DMF.

**15.** The liquid phase tide synthesis method according to any one of method clams 12 to 14, wherein the extraction solution is a reactive extraction solution for the reagents and optionally also for the by-products from the reaction solution, wherein the extraction solution is selected from an aqueous acid solution or ethanol.

**16.** Use of a solid phase extraction membrane (4) as herein provided in its different embodiments, in a liquid phase tide synthesis method and/or system.
